# EUROPEAN PATENT SPECIFICATION

(11) **EP 2 709 701 B1**
(45) Date of publication and mention of the grant of the patent: **18.11.2015**
(21) Application number: 12724769.0
(22) Date of filing: 16.05.2012
(51) Int. Cl.: A61M 15/02, B05B 5/025

(54) **SYSTEM FOR ELECTRO-HYDRODYNAMIC ATOMISATION**
SYSTEM FÜR ELEKTROHYDRODYNAMISCHE ZERSTÄUBUNG
SYSTÈME D'ATOMISATION ÉLECTRO-HYDRODYNAMIQUE

(30) Priority: 17.05.2011 NL 2006794
(43) Date of publication of application: 26.03.2014
(73) Proprietor: Gilbert Technologies B.V., 1411 DC Naarden (NL)
(72) Inventor: Marijnissen, Johannes Cornelis Maria, NL-4819 ED Breda (NL); Yurteri, Caner Umit, NL-2600 GA Delft (NL); Roos, Rein André, F-61340 Nocé (FR)
(74) Representative: Nederlandsch Octrooibureau
(86) International application number: PCT/NL2012/050341
(87) International publication number: WO 2012/158033

(56) References cited:
- WO-A2-2006/009854
- US-A1- 2003 209 005

## Description

### Field of the invention

The invention relates to a method and system for delivering particles via electrostatic spraying (electrospray) according to the features of the preamble of claim 1 or 16. A delivery device comprising these features is known from US 2003/0209 005.

### Background of the invention

Electro-hydrodynamic atomization (EHDA), or electrospraying, is a process where a liquid can be distributed into uniformly sized droplets or particles under the influence of an electrical force in a very controlled manner. EHDA may possess plenty of advantages in comparison to conventional systems, one of them being the relatively narrow size distribution of particles.

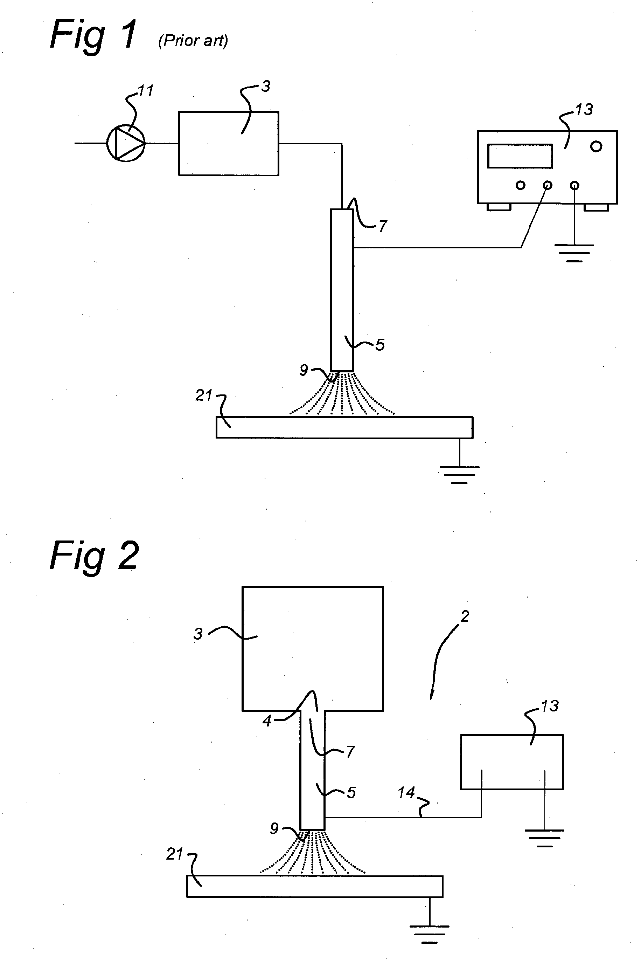

Figure 1 schematically shows a typical sprayed particle delivery system of the prior art.

In figure 1, the particle delivery system 1 comprises a reservoir 3 for holding a liquid substance, a nozzle 5 having an inlet 7 and an outlet 9 for releasing a spray of the liquid substance, a pump 2 for delivering the liquid substance from the reservoir to the nozzle, a counter electrode 21, a high voltage electric supply 13 for providing a high voltage electric field between the liquid at the outlet 9 of the nozzle 5 and the counter electrode 21.

The pump 2 is connected to the reservoir 3, and the reservoir is connected to the inlet 7 of the nozzle 5 via a conduit.

The pump 2 comprises a mechanical pump system 11 for pumping the liquid substance from the reservoir 3 to the nozzle 5. The pump system 11 is for example a piston pump or syringe pump.

The electric supply 13 is connected to the outlet 9 of the nozzle 5 via a conducting lead. The nozzle outlet 9 is located at some distance from the counter electrode 21 with the outlet 9 facing in the direction of the counter electrode 21. Both the counter electrode 21 and the electric supply 13 are connected to a common voltage level, e.g., ground voltage.

In the sprayed particles delivery device, the electric output provided by the electric supply 13 is typically in the range of nano-amperes at a high voltage (kV level) between the electrodes. The electric supply 13 provides an electric field between the nozzle and the counter electrode 21. The liquid substance fed from the pump 2 is introduced into the electric field at the outlet 9 of the nozzle 5. The electric field between the nozzle outlet 5 and the counter electrode 21 causes the liquid substance to be atomized and distributed into uniformly sized droplets or particles with a spatial distribution.

The abovementioned sprayed particle delivery system has some drawbacks:
- It is known that to obtain a constant flow of the liquid substance by a mechanical pump system may be difficult. Small variations of the flow of liquid substance may influence the size and distribution of the sprayed particles from the nozzle outlet.
- It is also known that a most constant flow is typically obtained by a syringe type of pump. This type of pump system has relatively large size and weight which negatively affects the portability of the particle delivery system.

### Summary of the invention

It is an object of the invention to reduce or mitigate the disadvantages from the prior art.

This object is achieved by a particle delivery device for producing a spray of particles in an ambient gas at an ambient pressure, comprising a storage volume for a liquid substance; a nozzle having an inlet and an outlet, the nozzle inlet being coupled to the storage volume in fluid communication; an electric supply for providing an electric field between the nozzle outlet and a counter electrode, wherein the storage volume is gastight, the storage volume is adapted to provide the liquid substance to the nozzle inlet at ambient pressure level, and the electric supply is arranged to control a release of the liquid substance from the nozzle outlet by a control of the electric field.

In this manner, no mechanical pump is required in the particle delivery device. The electric field generated between the liquid substance at the nozzle outlet and the counter electrode ejects the liquid substance in atomized form from the nozzle outlet which causes the electric supply to act as a pump since fresh liquid substance will be supplied into the nozzle from the storage volume.

By providing that the liquid substance in the storage volume is at ambient pressure, the back pressure during the spraying procedure remains constant. This ensures that the supply of liquid from the storage volume remains substantially in balance with respect to the amount of liquid ejected (i.e., the release or release rate) from the nozzle and virtually a zero underpressure is maintained.

Also, since the spraying rate (flow rate) is well controlled by the electric supply, the present invention simplifies the control procedure of the particle delivery device. As the flow rate has a relatively strong effect on the size of the particles produced, the control of the size of particles and the size distribution is enhanced. Additionally, the electric field as driving force can be kept substantially constant which results in a well controlled and constant flow rate.

Since as explained above, no mechanical pump is required in the particle delivery device, the particle delivery system has an advantage in that it can be relatively compact, even portable, due to the absence of such a mechanical pump.

The fact that the storage volume can adapt its shape as function of the consumption of the liquid allows the pressure of the liquid in the storage volume to remain equal to the pressure of the ambient. The ambient pressure may be any suitable pressure within a space in which the particle delivery device is installed. In an exemplary embodiment, the ambient pressure is atmospheric pressure.

According to an aspect of the invention, there is provided a particle delivery device as described above, wherein the nozzle is conductive.

The conductivity of the nozzle provides that the nozzle functions as an electrode to electrically charge the liquid substance.

According to an aspect of the invention, there is provided a particle delivery device as described above, wherein the nozzle is a capillary needle.

According to an aspect of the invention, there is provided a particle delivery device as described above, wherein the storage volume is a container with at least one moveable wall.

The moveable wall floating on the liquid substance provides that the liquid substance is substantially isolated from the ambient while the movement of the wall on top of the liquid allows the pressure of the liquid in the storage volume to remain equal to the pressure of the ambient.

According to an aspect of the invention, there is provided a particle delivery device as described above, wherein the storage volume is a container with at least one flexible wall.

According to an aspect of the invention, there is provided a particle delivery device as described above, wherein the container comprises side walls and the at least one moveable wall is adapted to move between the side walls in a direction parallel to the side walls.

According to an aspect of the invention, there is provided a particle delivery device as described above, wherein the storage volume is a plastic capsule having a foil wall.

A foil wall is known to have a virtually nonexistent strength under compression and will collapse (i.e. deform) until the external pressure is in equilibrium with the internal pressure in the capsule.

According to an aspect of the invention, there is provided a particle delivery device as described above, wherein the storage volume consists of a pouch or bag with a foil wall.

According to an aspect of the invention, there is provided a particle delivery device as described above, wherein the storage volume comprises two foils of substantially identical area size, wherein the two foils are joined together at edges so as to create the storage volume in between the two foils.

According to an aspect of the invention, there is provided a particle delivery device as described above, wherein one or more of the two foils comprises at least one layer selected from a metallic layer and a plastic layer.

According to an aspect of the invention, there is provided a particle delivery system as described above, wherein the capillary needle is dimensioned with an internal diameter and a length in such a way that the capillary needle locks the storage volume by means of the capillary force, if no electric field is present.

According to an aspect of the invention, there is provided a particle delivery device as described above, wherein the capillary needle further comprises a seal on its outlet.

Advantageously, the capillary needle can be kept sealed during pre-use, e.g. during transport or shelf storage.

According to an aspect of the invention, there is provided a particle delivery device as described above, wherein the internal diameter of the capillary needle is about 1 mm or less, preferably 250 micrometer or less, very preferably 100 micrometer or less.

Advantageously, the range of the internal capillary diameter is chosen such that in absence of an electric field, the capillary force allows the liquid to fill the volume of the capillary needle. The diameter of the capillary needle may depend on flow properties of the liquid substance, such as the viscosity. Also, the diameter may depend on the specific application of the particle delivery device.

According to an aspect of the invention, there is provided a particle delivery device as described above, wherein the length of the nozzle is around 5 mm, but other lengths are feasible depending on the application and the liquid substance that is to be atomized.

According to an aspect of the invention, there is provided a particle delivery device as described above, wherein the counter electrode is a ring shaped electrode spaced apart from the nozzle.

The ring shape of the counter electrode provides an aperture for a flow of sprayed particles generated on the nozzle outlet when an electric field between the nozzle outlet and the counter electrode is applied.

According to an aspect of the invention, there is provided a particle delivery device as described above, wherein the counter electrode is a mesh shaped electrode spaced apart from the nozzle.

The mesh shape of the counter electrode provides an aperture for a flow of sprayed particles generated on the nozzle outlet when an electric field between the nozzle outlet and the counter electrode is applied.

According to an aspect of the invention, there is provided an inhaler device comprising a particle delivery device as described above.

The particle delivery system of the present invention can be advantageously applied in portable applications for delivery of medical components into the respiratory airways of beings. Due to the absence of a pumping system the particle delivery system can be dimensioned with relative ease for incorporation into an inhaler device. Also, since no pumping system is required, the power supply can be relatively small and portable since it needs to deliver only power for the generation of the spray which is as mentioned above in the order of micro- to milliwatts.

According to an aspect of the invention, there is provided an inhaler device as described above, further comprising an inhaler mask wherein the nozzle outlet is arranged either in the mask or in an air inlet of the inhaler mask.

According to an aspect of the invention, there is provided a method for producing a spray of particles in an ambient gas at an ambient pressure, comprising
- providing a particle delivery device that comprises:
   a storage volume for a liquid substance; a nozzle having an inlet and an outlet, the nozzle inlet being connected to the storage volume; an electric supply for providing an electric field between the liquid substance at the nozzle outlet and a counter electrode;
   the method further comprising:
- providing the liquid substance in the storage volume, wherein the storage volume is gastight;
- creating the spray of particles at the nozzle outlet by the electric field,
- controlling a release of the liquid substance from the nozzle outlet by the electric supply using a control of the electric field and
- adapting the storage volume to provide the liquid substance at ambient pressure level at the nozzle inlet.

### Brief description of the drawings

The invention will be explained in detail with reference to some drawings that are only intended to show embodiments of the invention and not to limit the scope. The scope of the invention is defined in the annexed claims and by its technical equivalents.

The drawings show:
Figure 1 shows a schematic drawing of a prior art particles delivery system;
Figure 2 shows a schematic drawing of a particles delivery system according to an embodiment of the invention;
Figure 3 shows a schematic drawing of a particles delivery system according to an embodiment of the invention;
Figure 4 shows a schematic drawing of a particles delivery system according to an embodiment of the invention.

### Detailed description of embodiments

In figure 2, a particles delivery system according to an embodiment of the invention is shown. The particle delivering system 2 comprises a storage volume 3 for storing a liquid substance, a nozzle 5, having an inlet 7 and an outlet 9, a counter electrode 21, and a high voltage electric supply 13 for providing an electric field between the liquid substance at the outlet 9 of the capillary needle 5 and the counter electrode 21.

The storage volume 3 is connected to the inlet 7 of the nozzle 5 via a passage 4. The storage volume is gastight except for the passage 4 to the nozzle 5. The passage 4 sets up a connection between the nozzle 5 and the reservoir 3 for fluid communication between the reservoir and the nozzle.

The passage is not necessarily directly connected to the nozzle but optionally may comprise a tubing or conduit between the storage volume 3 and the nozzle 5 for feeding the liquid substance from the storage volume to the nozzle.

The electric supply 13 is connected to either an electrode (not shown) that contacts the liquid substance in the particle delivery device or alternatively if the nozzle is conductive, to the outlet 9 of the nozzle 5 via conducting leads 14.

Note that the electrode that contacts the liquid substance may be located in either the storage volume or the nozzle.

The conducting leads 14 are arranged to provide electric contact either to the liquid substance in the nozzle directly, or to the nozzle itself, in case the nozzle is a conductive nozzle.

The nozzle 5 is spaced apart from the counter electrode 21 with the nozzle outlet 9 facing towards the counter electrode 21. The nozzle outlet 9 is connected to one terminal of one polarity on the electric supply 13, the counter electrode 21 is connected to another terminal of opposite polarity on the electric supply.

In an embodiment, both the counter electrode 21 and the other terminal of the electric supply 13 are connected to a common voltage, e.g. ground voltage.

In the particles delivery system according to this embodiment, an electric supply 13 is arranged as feeding device by using a control of the electric field. The electric field generated between the nozzle outlet and the counter electrode ejects the liquid substance in atomized form from the nozzle outlet and acts as pump since fresh liquid substance will be supplied from the storage volume into the nozzle.

Additionally, the storage volume 3 is adapted to provide the liquid substance to the nozzle inlet at ambient pressure level, basically by adapting its shape in such a way that the inner pressure in the storage volume is substantially equal to the outer ambient pressure. Due to the substantially constant pressure in the storage volume 3, the influence of the pressure difference on the liquid flow rate from the storage volume 3 has been substantially eliminated.

In the particle delivery system, the electric field thus becomes a major driving force for transport of the liquid substance in the nozzle by spraying the liquid substance out from the nozzle 5.
Further, by providing that the pressure of the liquid substance is constant, fluctuations in the flow rate of the liquid can be reduced. Additionally, the electric field can be controlled so as to tune the size of the particles in the spray as substantially constant in time. As a result, a spray of fine and uniformly sized particles can be produced over relatively long periods of time.

The electric output provided by the electric supply 13 is in the range of nano-amperes at a high voltage (kV level). The delivered liquid is atomized and sprayed by the electric field provided between the nozzle 5 and the counter electrode 21.

In an embodiment, the counter electrode 21 has apertures through which the spray may pass into the ambient.

In an embodiment, the counter electrode 21 may be a ring shaped object or a mesh, spaced apart from the nozzle 5.

Other shapes of the counter electrode are conceivable depending on the specific utilization of the particle delivery system.

It will be appreciated that the nozzle outlet 9 can be positioned in any direction with respect to the surface level of the liquid substance in the storage volume.

In an embodiment, the nozzle is a capillary needle. By use of a capillary needle as nozzle, the liquid substance can be transported from the storage volume to the nozzle outlet using the capillary force in the needle.

Additionally, the capillary needle can be dimensioned in such a way that when no (high voltage) electric field is present at the nozzle outlet, the capillary forces block an uncontrolled outflow of the liquid substance and the capillary needle functions as a capillary lock.

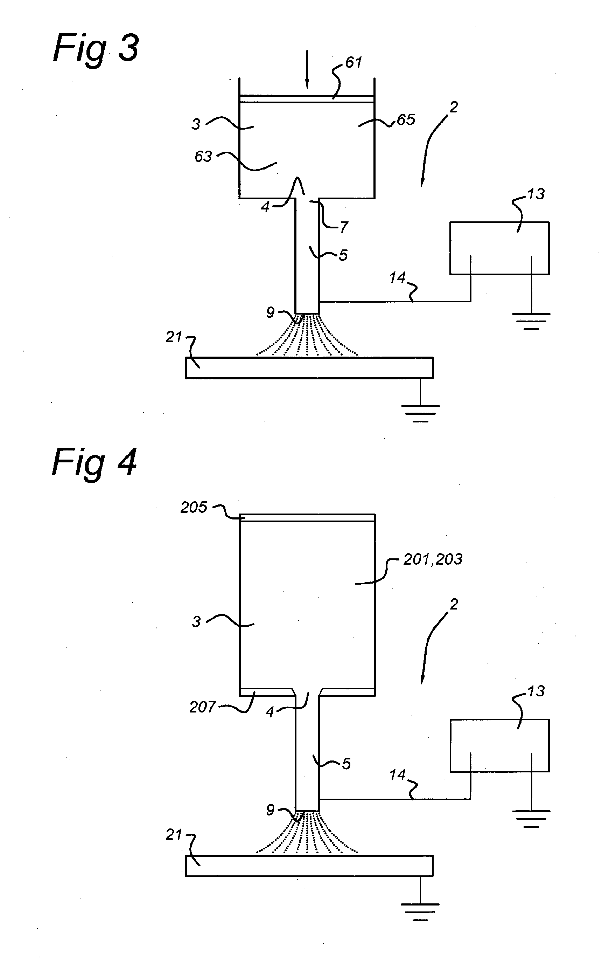

Figure 3 shows a schematic drawing of a particles delivery system according to an embodiment of the invention. In figure 3, the storage volume 3 is a container with at least one moveable wall which by its movement provides that the storage volume is capable to provide the liquid substance to the nozzle inlet 7 at ambient pressure level.

When the storage volume 3 is filled with a liquid substance, the moveable wall is arranged to float on the liquid substance. Next, when the liquid substance is sprayed from the nozzle outlet 9, the moveable wall 61 is free to move and follow the level of the liquid in the storage volume 3 to achieve that the pressure in the storage volume is substantially equal to the ambient pressure.

In an embodiment, the storage volume 3 comprises parallel side walls 63, 65 and the at least one moveable wall 61 is adapted to move between the side walls 63, 65 in a direction parallel to the side walls 63, 65.

In an alternative embodiment, the storage volume 3 comprises parallel side walls 63, 65 and at least one flexible wall 61 that is adapted to move to adapt the volume of the storage volume.

Figure 4 shows a schematic drawing of a particles delivery system according to an embodiment of the invention.

In this embodiment of the particles delivery system, the storage volume 3 is arranged to dynamically adapt the shape of the storage volume based on an externally exerted pressure on the storage volume 3.

The storage volume 3 is constructed in a manner that when an amount of the liquid substance is taken out of the storage volume, the storage volume adapts its shape and reduces the inner volume of the storage volume accordingly, in such a way that the liquid substance in the storage volume remains at ambient pressure.

In this manner, the inner volume is reduced by the same amount as the liquid taken out, so as to balance the ambient pressure with the inner pressure of the storage volume.

The construction of the storage volume may comprise one or more walls that consist of foil materials. Examples of such storage volumes according to the present invention, comprise a bag or pouch with foil walls. The use of foil walls allows that the storage volume can collapse as a result of the outflow of the sprayed liquid substance as driven by the electric field.

The storage volume 3 comprises foil parts 201, 203 of substantially identical area size.

The two foil parts 201, 203 are joined together on their edges (e.g. by a weld) so as to create the storage volume between the two foils 201, 203. A passage opening 4 is arranged on one of the edges, or in one of the foil parts. The storage volume is gastight except for the passage 4.

The foil(s) comprises at least one layer selected from a metallic layer and a plastic layer.

The foil material may comprise a plastic foil, e.g. polyethylene or polypropylene, or a metallic foil such as aluminum. Additionally, the foil material may comprise a plastic foil coated with a thin film coating comprising a metal.

Moreover, the foil(s) may consist two or more foil layers laminated to each other.

Alternatively, the storage volume may comprise a blown extruded plastic foil with circular cross section with joined upper and lower edges 205, 207. In yet another embodiment of the invention, the storage volume 3 is a plastic capsule having foil walls with a passage 4 for coupling to the nozzle 5.

Advantageously, the particle delivery system can be arranged within an inhaler device for administering sprayed liquid substance to a being.

The outlet 9 of the capillary needle 5 and the counter electrode 21 are positioned within the inhaler that is intended to be directed to the mouth and/or nose of the being, such that during use a spray generated at the capillary outlet can be directed into the airways of the being.

In a further embodiment the inhaler comprises an inhaler mask. which can be placed over the airways (mouth and/or nose) of a being that needs to be administered with a sprayed liquid substance. Since the particle delivery device is capable of an enhanced control of particle sizes and size distribution, the inhaler device advantageously allows that based on the produced particle size, particles of a sprayed liquid substance can be directed selectively to a specific region of the respiratory system.

In an embodiment of the particle delivery device, the capillary needle 5 further comprises a seal (not shown) on the capillary needle 5. The seal can provide a secure delivery of the liquid substance preventing exposure of the liquid to the ambient. Advantageously, the capillary needle 5 can be kept sealed e.g. during shelf storage or transport.

The range of the capillary needle diameter and/or length may be chosen, individually or in combination, in dependence of the properties of the liquid substance needed to be delivered as a spray. The properties of the liquid substance may include the viscosity, the density, the composition, etc.

Without any limitation of the invention the following examples for capillary needles are given. a suitable length of the capillary needle 5 can be around 5 mm An inner diameter of the capillary needle 5 is depending on the application and/or liquid substance to be electrosprayed and can be about 1 mm or less, or about 500 micrometer, or about 250 micrometer or less, or about 100 micrometer or less. As a further example, for production of particles for inhalation purposes a 30 gauge needle may be used as capillary needle. It has an inner diameter of 0.150 mm and is 6.35 mm in length.

The present invention also relates to a method for producing a spray of particles in an ambient gas at an ambient pressure, comprising
- providing a particle delivery device that comprises:
   a storage volume 3 for a liquid substance; a nozzle 5 having an inlet 7 and an outlet 9, the nozzle inlet 7 being coupled to the storage volume 3 in fluid communication; an electric supply 13 for providing an electric field between the nozzle outlet 9 and a counter electrode 21,
   the method further comprising:
- providing the liquid substance in the storage volume, wherein the storage volume is a gastight volume;
- creating the spray of particles by the electric field,
- controlling a release of the liquid substance from the storage volume 3 to the nozzle outlet by the electric supply 13 using a control of the electric field and
- adapting the storage volume to provide the liquid substance at ambient pressure level at the nozzle inlet.

The description is intended only to illustrate the invention. The examples described hereinabove are not intended to limit the scope of the invention, which is defined by the appended claims.

The present invention can be described by the following embodiments:
Embodiment 1. A particle delivery device for producing a spray of particles in an ambient gas at an ambient pressure, comprising a storage volume (2) for a liquid substance; a nozzle (5) having an inlet (7) and an outlet (9), the nozzle inlet (7) being coupled to the storage volume (2) in fluid communication; an electric supply (13) for providing an electric field between the liquid substance at the nozzle outlet (9) and a counter electrode (21), wherein the storage volume is gastight, the storage volume is a container with at least one moveable wall which is adapted to provide the liquid substance to the nozzle inlet at ambient pressure level, and the electric supply (13) is arranged to control a release rate of the liquid substance from the nozzle outlet by a control of the electric field, and wherein the liquid substance in the storage volume is at ambient pressure.
Embodiment 2. The particle device according to embodiment 1, further comprising an electrode for contacting the liquid substance, and wherein the electric supply is connected to the electrode.
Embodiment 3. The particle delivery device according to embodiment 2, wherein the electrode is located in the nozzle.
Embodiment 4. The particle delivery device according to embodiment 2, wherein the electrode is located in the storage volume.
Embodiment 5. The particle delivery device according to embodiment 1, wherein the nozzle is conductive, and the electric supply connects to the nozzle.
Embodiment 6. The particle delivery device according to any one of the preceding embodiments, wherein the nozzle is a capillary needle.
Embodiment 7. The particle delivery device according to embodiment 1, wherein the storage volume is a container with at least one moveable wall.
Embodiment 8. The particle device according to embodiment 7, wherein the container comprises side walls and the at least one moveable wall is adapted to move between the side walls in a direction parallel to the side walls.
Embodiment 9. The particle delivery device according to embodiment 1, wherein the storage volume is a container with at least one flexible wall.
Embodiment 10. The particle delivery system according to embodiment 1, wherein the storage volume is arranged to adapt a shape of the storage volume based on a difference between the pressure in the storage volume and the ambient pressure.
Embodiment 11. The particle delivery device according to any one of the preceding embodiments 1 - 10, wherein the storage volume (2) is a plastic capsule having a foil wall.
Embodiment 12. The particle delivery device according to any one of the preceding embodiments 1 - 10, wherein the storage volume (2) comprises two foils (201, 203) of substantially identical area size, wherein the foils are joined together at edges of the foils so as to create the storage volume in-between the two foils.
Embodiment 13. The particle delivery device according to embodiment 11 or embodiment 12, wherein the foil comprises at least one layer selected from a metallic layer and a plastic layer.
Embodiment 14. The particle delivery system according to any one of the preceding embodiments 6 - 13, wherein the capillary needle is dimensioned with a diameter and a length in such a way that the capillary needle locks the storage volume by means of the capillary force, if no electric field is supplied.
Embodiment 15. The particle delivery system according to any one of the preceding embodiments 6 - 14, wherein the capillary needle further comprises a seal on its outlet.
Embodiment 16. The particle delivery system according to any one of the preceding embodiments 6 - 15, wherein the diameter of the capillary needle is 1 mm or less; preferably 250 micrometer or less; more preferably 100 micrometer or less.
Embodiment 17. The particle delivery system according to any one of the preceding embodiments 6 - 16, wherein the length of the nozzle is around 5 mm.
Embodiment 18. The particle delivery device according to any one of the preceding embodiments, wherein the counter electrode is a ring shaped electrode spaced apart from the nozzle.
Embodiment 19. The particle delivery device according to any one of the preceding embodiments 1 - 18, wherein the counter electrode is a mesh electrode spaced apart from the nozzle.
Embodiment 20. An inhaler device comprising a particle delivery device according to any one of the preceding embodiments 1 - 19.
Embodiment 21. The inhaler device according to embodiment 20, further comprising an inhaler mask wherein the nozzle outlet is arranged either in the mask or in an air inlet of the inhaler mask.
Embodiment 22. A method for producing a spray of particles in an ambient gas at an ambient pressure, comprising - providing a particle delivery device that comprises: a storage volume (2) for a liquid substance; a nozzle (5) having an inlet (7) and an outlet (9), the nozzle inlet (7) being coupled to the storage volume (2) in fluid communication; an electric supply (13) for providing an electric field between the nozzle outlet (9) and a counter electrode (21); the method further comprising:
   - providing the liquid substance in the storage volume, wherein the storage volume is gastight; - creating the spray of particles at the nozzle outlet by the electric field,
   - controlling a release of the liquid substance from the nozzle outlet (9) by the electric supply (13) using a control of the electric field and - adapting the storage volume to provide the liquid substance at ambient pressure level at the nozzle inlet.

## Claims

1. A particle delivery device for producing a spray of particles in an ambient gas at an ambient pressure, comprising
a storage volume (2) for a liquid substance;
a nozzle (5) having an inlet (7) and an outlet (9), the nozzle inlet (7) being coupled to the storage volume (2) in fluid communication;
an electric supply (13) for providing an electric field between the liquid substance at the nozzle outlet (9) and a counter electrode (21),
wherein the storage volume is gastight, wherein the electric supply (13) is arranged to control a release rate of the liquid substance from the nozzle outlet by a control of the electric field **characterised in that** the storage volume is a container with at least one moveable wall which is adapted to provide the liquid substance to the nozzle inlet at ambient pressure level, and
wherein the liquid substance in the storage volume is at ambient pressure.

2. The particle device according to claim 1, further comprising an electrode for contacting the liquid substance, and wherein the electric supply is connected to the electrode.

3. The particle delivery device according to claim 2, wherein the electrode is located in the nozzle.

4. The particle delivery device according to claim 2, wherein the electrode is located in the storage volume.

5. The particle delivery device according to claim 1, wherein the nozzle is conductive, and the electric supply connects to the nozzle.

6. The particle delivery device according to any one of the preceding claims, wherein the nozzle is a capillary needle, wherein the capillary needle is dimensioned with a diameter and a length in such a way that the capillary needle locks the storage volume by means of the capillary force, if no electric field is supplied..

7. The particle delivery device according to claim 1, wherein the storage volume is a container with at least one moveable wall.

8. The particle device according to claim 7, wherein the container comprises side walls and the at least one moveable wall is adapted to move between the side walls in a direction parallel to the side walls.

9. The particle delivery device according to claim 1, wherein the storage volume is a container with at least one flexible wall.

10. The particle delivery system according to claim 1, wherein the storage volume is arranged to adapt a shape of the storage volume based on a difference between the pressure in the storage volume and the ambient pressure.

11. The particle delivery device according to any one of claims 1 - 10, wherein the storage volume (2) is a plastic capsule having a foil wall.

12. The particle delivery device according to any one of claims 1 - 10, wherein the storage volume (2) comprises two foils (201, 203) of substantially identical area size, wherein the foils are joined together at edges of the foils so as to create the storage volume in-between the two foils.

13. The particle delivery device according to any one of the preceding claims, wherein the counter electrode is a ring shaped electrode spaced apart from the nozzle.

14. The particle delivery device according to any one of the preceding claims 1 - 13, wherein the counter electrode is a mesh electrode spaced apart from the nozzle.

15. An inhaler device comprising a particle delivery device according to any one of the preceding claims 1 - 14.

16. A method for producing a spray of particles in an ambient gas at an ambient pressure, comprising
- providing a particle delivery device that comprises:
a storage volume (2) for a liquid substance; a nozzle (5) having an inlet (7) and
an outlet (9), the nozzle inlet (7) being coupled to the storage volume (2) in fluid communication; an electric supply (13) for providing an electric field between the nozzle outlet (9) and a counter electrode (21);
the method further comprising:
- providing the liquid substance in the storage volume, wherein the storage volume is gastight;
- creating the spray of particles at the nozzle outlet by the electric field,
- controlling a release of the liquid substance from the nozzle outlet (9) by the electric supply (13) using a control of the electric field **characterised by**
- adapting the storage volume to provide the liquid substance at ambient pressure level at the nozzle inlet.

## Patentansprüche

1. Eine Partikelliefereinrichtung zum Erzeugen eines Sprühnebels von Partikeln in einem Umgebungsgas bei einem Umgebungsdruck, die Folgendes aufweist:
ein Speichervolumen (2) für eine flüssige Substanz;
eine Düse (5) mit einem Einlass (7) und einem Auslass (9), wobei der Düseneinlass (7) an das Speichervolumen (2) in Strömungsmittelverbindung gekoppelt ist;
eine elektrische Versorgung (13), um ein elektrisches Feld zwischen der flüssigen Substanz an dem Düsenauslass (9) und einer Gegenelektrode (21) vorzusehen,
wobei das Speichervolumen gasdicht ist, wobei die elektrische Versorgung (13) angeordnet ist, um eine Abgaberate der flüssigen Substanz von dem Düsenauslass durch eine Steuerung des elektrischen Feldes zu steuern,
**dadurch gekennzeichnet, dass** das Speichervolumen ein Behälter mit wenigstens einer bewegbaren Wand ist, die ausgelegt ist, um die flüssige Substanz an den Düseneinlass bei einem Umgebungsdruckpegel zu liefern, und
wobei die flüssige Substanz in dem Speichervolumen auf Umgebungsdruck ist.

2. Partikelliefereinrichtung nach Anspruch 1, die weiter eine Elektrode zum Kontaktieren der flüssigen Substanz aufweist, und wobei die elektrische Versorgung mit der Elektrode verbunden ist.

3. Partikelliefereinrichtung nach Anspruch 2, wobei die Elektrode in der Düse angeordnet ist.

4. Partikelliefereinrichtung nach Anspruch 2, wobei die Elektrode in dem Speichervolumen angeordnet ist.

5. Partikelliefereinrichtung nach Anspruch 1, wobei die Düse leitend ist, und wobei die elektrische Versorgung mit der Düse verbunden ist.

6. Partikelliefereinrichtung nach einem der vorhergehenden Ansprüche, wobei die Düse eine Kapillarnadel ist, wobei die Kapillarnadel mit einem Durchmesser und einer Länge auf solche Weise bemessen ist, dass die Kapillarnadel das Speichervolumen mittels der Kapillarkraft verschließt, wenn kein elektrisches Feld angelegt wird.

7. Partikelliefereinrichtung nach Anspruch 1, wobei das Speichervolumen ein Behälter mit wenigstens einer bewegbaren Wand ist.

8. Partikelliefereinrichtung nach Anspruch 7, wobei der Behälter Seitenwände aufweist und die wenigstens eine bewegbare Wand ausgelegt ist, um sich zwischen den Seitenwänden in einer Richtung parallel zu den Seitenwänden zu bewegen.

9. Partikelliefereinrichtung nach Anspruch 1, wobei das Speichervolumen ein Behälter mit wenigstens einer flexiblen Wand ist.

10. Partikelliefereinrichtung nach Anspruch 1, wobei das Speichervolumen angeordnet ist, um eine Form des Speichervolumens anzunehmen, die auf einer Differenz zwischen dem Druck in dem Speichervolumen und dem Umgebungsdruck basiert.

11. Partikelliefereinrichtung nach einem der Ansprüche 1 - 10, wobei das Speichervolumen (2) eine Plastikkapsel ist, die eine Wand aus Folie hat.

12. Partikelliefereinrichtung nach einem der Ansprüche 1 - 10, wobei das Speichervolumen (2) zwei Folien (201, 203) aufweist, die eine im Wesentlichen identische Flächenabmessung haben, wobei die Folien an den Kanten der Folien verbunden sind, um so das Speichervolumen zwischen den beiden Folien zu bilden.

13. Partikelliefereinrichtung nach einem der vorhergehenden Ansprüche, wobei die Gegenelektrode eine ringförmige Elektrode ist, die von der Düse beabstandet ist.

14. Partikelliefereinrichtung nach einem der vorhergehenden Ansprüche 1 - 13, wobei die Gegenelektrode eine Gitterelektrode ist, die von der Düse beabstandet ist.

15. Inhalationseinrichtung, die eine Partikelliefereinrichtung gemäß einem der vorhergehenden Ansprüche 1 - 14 aufweist.

16. Ein Verfahren zum Erzeugen eines Sprühnebels von Partikeln in einem Umgebungsgas bei einem Umgebungsdruck, das Folgendes aufweist:
Vorsehen einer Partikelliefereinrichtung, die Folgendes aufweist:
ein Speichervolumen (2) für eine flüssige Substanz; eine Düse (5) mit einem Einlass (7) und einem Auslass (9), wobei der Düseneinlass (7) an das Speichervolumen (2) in Strömungsmittelverbindung gekoppelt ist; eine elektrische Versorgung (13), um ein elektrisches Feld zwischen dem Düsenauslass (9) und einer Gegenelektrode (21) vorzusehen;
wobei das Verfahren weiter Folgendes aufweist:
Liefern der flüssigen Substanz in das Speichervolumen, wobei das Speichervolumen gasdicht ist;
Erzeugen eines Sprühnebels von Partikeln an dem Düsenauslass durch das elektrische Feld,
Steuern einer Abgabe der flüssigen Substanz von dem Düsenauslass (9) durch die elektrische Versorgung (13) unter Verwendung einer Steuerung des elektrischen Feldes,
**gekennzeichnet durch**
Anpassen des Speichervolumens, um die flüssige Substanz bei Umgebungsdruck am Düseneinlass zu liefern.

## Revendications

1. Dispositif de délivrance de particules pour produire une pulvérisation de particules dans un gaz ambiant à pression ambiante, comprenant
un volume de stockage (2) pour une substance liquide ;
une buse (5) ayant une entrée (7) et une sortie (9), l'entrée de buse (7) étant couplée au volume de stockage (2) en communication fluidique ;
une alimentation électrique (13) pour fournir un champ électrique entre la substance liquide au niveau de la sortie de buse (9) et une contre-électrode (21),
dans lequel le volume de stockage est étanche aux gaz,
dans lequel l'alimentation électrique (13) est agencé pour commander une vitesse de libération de la substance liquide à partir de la sortie de buse par une commande du champ électrique,
**caractérisé en ce que**
le volume de stockage est un récipient avec au moins une paroi mobile qui est adaptée pour fournir la substance liquide à l'entrée de buse au niveau de pression ambiante, et
dans lequel la substance liquide dans le volume de stockage est à pression ambiante.

2. Dispositif à particules selon la revendication 1, comprenant en outre une électrode pour venir en contact avec la substance liquide, et dans lequel l'alimentation électrique est reliée à l'électrode.

3. Dispositif de délivrance de particules selon la revendication 2, dans lequel l'électrode est située dans la buse.

4. Dispositif de délivrance de particules selon la revendication 2, dans lequel l'électrode est située dans le volume de stockage.

5. Dispositif de délivrance de particules selon la revendication 1, dans lequel la buse est conductrice et l'alimentation électrique est reliée à la buse.

6. Dispositif de délivrance de particules selon l'une quelconque des revendications précédentes, dans lequel la buse est une aiguille capillaire, où l'aiguille capillaire est dimensionnée avec un diamètre et une longueur de sorte que l'aiguille capillaire verrouille le volume de stockage au moyen de la force capillaire, si aucun champ électrique n'est fourni.

7. Dispositif de délivrance de particules selon la revendication 1, dans lequel le volume de stockage est un récipient avec au moins une paroi mobile.

8. Dispositif à particules selon la revendication 7, dans lequel le récipient comprend des parois latérales et l'au moins une paroi mobile est adaptée pour se déplacer entre les parois latérales dans une direction parallèle aux parois latérales.

9. Dispositif de délivrance de particules selon la revendication 1, dans lequel le volume de stockage est un récipient avec au moins une paroi flexible.

10. Dispositif de délivrance de particules selon la revendication 1, dans lequel le volume de stockage est agencé pour adapter une forme du volume de stockage sur la base d'une différence entre la pression dans le volume de stockage et la pression ambiante.

11. Dispositif de délivrance de particules selon l'une quelconque des revendications 1 à 10, dans lequel le volume de stockage (2) est une capsule en plastique ayant une paroi en feuille.

12. Dispositif de délivrance de particules selon l'une quelconque des revendications 1 à 10, dans lequel le volume de stockage (2) comprend deux feuilles (201, 203) de superficie essentiellement identique, dans lequel les feuilles sont assemblées au niveau des bords des feuilles de manière à créer le volume de stockage entre les deux feuilles.

13. Dispositif de délivrance de particules selon l'une quelconque des revendications précédentes, dans lequel la contre-électrode est une électrode de forme annulaire espacée de la buse.

14. Dispositif de délivrance de particules selon l'une quelconque des revendications précédentes 1 à 13, dans lequel la contre-électrode est une électrode à mailles espacée de la buse.

15. Dispositif inhalateur comprenant un dispositif de délivrance de particules selon l'une quelconque des revendications précédentes 1 à 14.

16. Procédé de production d'une pulvérisation de particules dans un gaz ambiant à pression ambiante, comprenant le fait
- de fournir un dispositif de délivrance de particules qui comprend :
un volume de stockage (2) pour une substance liquide ; une buse (5) ayant une entrée (7) et une sortie (9), l'entrée de buse (7) étant couplée au volume de stockage (2) en communication fluidique ; une alimentation électrique (13) pour fournir un champ électrique entre la sortie de buse (9) et une contre-électrode (21) ;
le procédé comprenant en outre le fait :
- de fournir la substance liquide dans le volume de stockage, où le volume de stockage est étanche aux gaz ;
- de créer la pulvérisation de particules au niveau de la sortie de buse par le champ électrique,
- de commander une libération de la substance liquide à partir de la sortie de buse (9) par l'alimentation électrique (13) en utilisant une commande du champ électrique, **caractérisé par** le fait
- d'adapter le volume de stockage pour fournir la substance liquide au niveau de pression ambiante au niveau de l'entrée de buse.
